(19) Europäisches Patentamt
European Patent Office
Office européen des brevets

(11) Veröffentlichungsnummer: **0 061 168**
**B1**

(12) # EUROPÄISCHE PATENTSCHRIFT

(45) Veröffentlichungstag der Patentschrift: **30.05.84**

(51) Int. Cl.³: **C 07 C 85/26,** C 07 C 87/50

(21) Anmeldenummer: **82102258.9**

(22) Anmeldetag: **19.03.82**

(54) **Verfahren zur Reinigung von rohem 3,4,3',4'-Tetraaminodiphenyl.**

(30) Priorität: **24.03.81 DE 3111470**

(43) Veröffentlichungstag der Anmeldung:
**29.09.82 Patentblatt 82/39**

(45) Bekanntmachung des Hinweises auf die Patenterteilung:
**30.05.84 Patentblatt 84/22**

(84) Benannte Vertragsstaaten:
**BE CH DE FR GB IT LI NL**

(56) Entgegenhaltungen:
**US - A - 3 255 356**
**US - A - 3 865 876**
**US - A - 3 943 175**
**US - A - 3 975 387**

(73) Patentinhaber: **HOECHST AKTIENGESELLSCHAFT, Postfach 80 03 20, D-6230 Frankfurt am Main 80 (DE)**

(72) Erfinder: **Schubert, Hans, Dr., Hölderlinstrasse 56, D-6233 Kelkheim (Taunus) (DE)**
Erfinder: **Baessler, Konrad, Dr., Drosselweg 1, D-6230 Frankfurt am Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

3,4,3',4'-Tetraaminodiphenyl – im folgenden mit TAD abgekürzt – ist ein wertvolles Zwischen- und Endprodukt auf verschiedenen Sachgebieten. Als Zwischenprodukt wird TAD z. B. eingesetzt zur Herstellung von hochtemperaturbeständigen Polymeren wie beispielsweise von Poly-2,2'-(m-phenylen)-5,5'-bibenzimidazol (vgl. US-PSen 2 895 948 und 3 174 947); Endprodukt ist TAD z.B. bei seiner Verwendung als Antioxidans und als Mittel zur Stabilisierung von Epoxidharzen.

Zur Herstellung von TAD sind verschiedene Methoden bekannt. Eine derartige bekannte Methode ist die Ammonolyse von 3,3'-Dichlorbenzidin – im folgenden mit DCB abgekürzt – in Gegenwart hauptsächlich von Cu-Katalysatoren (sowohl Cu-Verbindungen als auch elementares Cu):

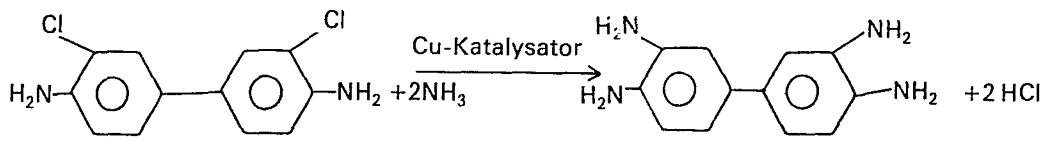


DCB TAD

Die Ammonolyse wird im allgemeinen hier mittels einer wässrigen $NH_3$-Lösung durchgeführt.

Eine derartige Ammonolyse von 3,3'-Dihalogenbenzidinen, bevorzugt DCB, in Gegenwart eines Cu-I-Salzes und/oder von $Cu_2O$ sowie eines Erdalkalisalzes (vorzugsweise $CaCl_2$) bei erhöhter Temperatur (100 bis 250°C, bevorzugt 150–210°C) und erhöhtem Inertgasdruck ist beschrieben in der FR-PS 1 475 631. Das so erhaltene rohe TAD wird über sein Salz mit einer starken Säure gereinigt. Die Ausbeuteangaben liegen bei 70% d.Th.

Wie die in den beiden US-PSen 3 865 876 und 3 943 175 enthaltenen Vergleichsbeispiele (Example 3 bzw. 7) zeigen, liefert das Verfahren der vorgenannten FR-PS vor der Reinigung über ein entsprechendes Salz eine Ausbeute an rohem TAD von etwa 82% (= 202 g) bei einer Reinheit des Produktes von 82% (laut Vergleichsbeispiel = Example 3 der US-PS 3 865 876); aus diesem Rohprodukt soll dann über das HCl-Salz mit nachfolgender Neutralisation mit NaOH 62,9% eines noch erhebliche Mengen an Cl und Cu enthaltenden TAD erhalten werden (laut Vergleichsbeispiel = Example 7 der US-PS 3 943 175).

Eine Verbesserung dieses wenig befriedigenden Ergebnisses der Methode gemäss der vorerwähnten FR-PS soll nach dem Verfahren der US-PS 3 865 876 dadurch erreicht werden, dass bei der Ammonolyse von DCB als Katalysator im wesentlichen nur Cu-I-chlorid verwendet wird. Die Ausbeuteangaben liegen hier bei etwa 75 bis 82% d.Th. eines Produktes mit einer Reinheit zwischen etwa 85 und 87%.

Dieses Produkt, dessen Cu-Gehalt etwa 3 bis 6 (Gewichts-)% beträgt, soll sich nach dem Verfahren der US-PS 3 943 175 (gemäss dessen Beispielen neben Cu-I-chlorid auch Cu-I-chlorid/Cu-Pulver als Katalysator verwendet werden kann) vorteilhaft über das Sulfat (Fällung des TAD-Sulfats mit Schwefelsäure, Abtrennung des Sulfats und Freisetzung des TAD daraus mit einer Base) mit gegebenenfalls nachfolgendem Umlösen aus wässriger Lösung – vorzugsweise unter Zusatz von Absorbentien wie z.B. von Aktivkohle und von Diatomeenerde – erreichen lassen. Nach den Angaben dieser US-PS beträgt der Cu-Gehalt

setzung mit einer Base etwa 0,6 bis 0,9%, nach dem sich noch daran anschliessenden Umlösen etwa 0,01 bis 0,07%. Die Ausbeute an so gereinigtem TAD liegt hier maximal bei 51,9%, bezogen auf das eingesetzte Roh-TAD (vgl. Tabelle 2, Run 6), bzw. maximal bei 45,7% d.Th., bezogen auf eingesetztes DCB (berechnet aus den angegebenen Werten).

Ähnlich wie die TAD-Herstellung und -Reinigung gemäss den US-PSen 3 865 876 und 3 943 175 verläuft auch die Ammonolyse von DCB in Gegenwart von elementarem Cu oder eines Cu-Oxids oder -Salzes mit nachfolgender Reinigung über ein Salz einer anorganischen Säure gemäss den JP-ASen 74-11212 und 74-11213.

Wegen der beträchtlichen Substanzverluste bei den bekannten Verfahren der Reinigung von Roh-TAD sowie auch wegen des mit der Durchführung dieser Verfahren verbundenen nicht unerheblichen Aufwands bestand die Aufgabe, diese Verfahren zu verbessern oder ein neues, verbessertes Reinigungsverfahren zu entwickeln.

Diese Aufgabe konnte erfindungsgemäss dadurch gelöst werden, dass das Cu-haltige Roh-TAD nach seiner Isolierung aus dem Herstellungsprozess mit einer wässrigen $NH_3$-Lösung behandelt und dass – falls man das so behandelte TAD in Gegenwart von Absorbentien noch aus wässriger Lösung umlöst – dabei ein wasserlösliches Reduktionsmittel zugesetzt wird.

Erfindungsgegenstand ist somit ein Verfahren zur Reinigung von rohem TAD, welches durch Ammonolyse von DCB in Gegenwart hauptsächlich von Cu-Katalysatoren hergestellt wurde,

a) durch Behandlung des rohen TAD mit einer wässrigen Lösung sowie gegebenenfalls
b) Umlösen des so behandelten TAD aus Wasser in Gegenwart von Absorbentien;

das Verfahren ist dadurch gekennzeichnet, dass man

a) das rohe TAD nach seiner Isolierung aus dem Herstellungsprozess mit einer wässrigen $NH_3$-Lö-
des TAD nach der Fällung als Sulfat und der Freisung behandelt und dass man
b) beim Umlösen des so behandelten TAD aus Wasser in Gegenwart von Absorbentien noch ein wasserlösliches Reduktionsmittel zusetzt.

Nach diesem Verfahren wird - ausgehend von einem Roh-TAD mit einem Cu-Gehalt zwischen etwa 3 und 6 (Gew.-)% - in dem gereinigten Endprodukt mindestens etwa der gleiche Cu-Gehalt erreicht wie nach den Verfahren der US-PS 3 943 175 (nach Stufe a ≦ 0,6 bis etwa 0,9% Cu, nach der zusätzlichen Stufe b ≦ 0,01 bis etwa 0,07% Cu); nach dem erfindungsgemässen Reinigungsverfahren liegt die Ausbeute an reinem TAD nach den beiden Reinigungsstufen jedoch etwa 25% höher (etwa bei 77%) als bei dem Verfahren der genannten US-PS (maximal 51,9%), bezogen auf das eingesetzte Roh-TAD. Ausserdem ist die erfindungsgemässe Behandlung des rohen TAD mit einer wässrigen $NH_3$-Lösung erheblich einfacher durchführbar als etwa die Ausfällung des TAD mit einer starken Mineralsäure mit nachfolgender Basenbehandlung nach dem Stand der Technik. Der Zusatz eines wasserlöslichen Reduktionsmittel in Stufe b) des erfindungsgemässen Verfahrens führt insbesondere zu einem helleren Produkt als nur die Umlösung aus Wasser in Gegenwart von Adsorbentien ohne ein wasserlösliches Reduktionsmittel.

Die Durchführung und der Erfolg vor allem von Stufe a) des erfindungsgemässen Verfahrens waren durch nichts nahegelegt und ausserordentlich überraschend. Die Senkung des Cu-Gehaltes des Roh-TAD in dieser Verfahrensstufe beruht wahrscheinlich auf einem Herauslösen von Cu-Verbindungen in komplexer Form mit $NH_3$. Da jedoch bei der Herstellung des TAD durch Ammonolyse von DCB mit nicht unerheblichen Mengen überschüssiger konzentrierter wässriger $NH_3$-Lösung gearbeitet wird, war anzunehmen, dass damit alle in lösliche Cu-$NH_3$-Komplexverbindungen überführbare Cu-Verbindungen in solche löslichen Komplexverbindungen überführt und nach der Abtrennung des erhaltenen Roh-TAD mit Wasser ausgewaschen werden. Dass eine Behandlung des nach dem Herstellungsprozess abgetrennten Roh-TAD mit einer wässrigen $NH_{-3}$-Lösung dennoch eine drastische Erniedrigung des Cu-Gehaltes zur Folge hat, war hier in keiner Weise mehr zu erwarten.

Auch die Farbverbesserung des Produktes durch den Zusatz eines wasserlöslichen Reduktionsmittels in Stufe b) war nicht mehr zu erwarten, da man annehmen konnte, dass durch die Gegenwart der Adsorbentien praktisch alle für Verfärbungen verantwortlichen Stoffe entfernt werden.

Das für den erfindungsgemässe Reinigungsverfahren als Ausgangsmaterial eingesetzte Roh-TAD ist das Produkt, welches durch Ammonolyse von DCB in Gegenwart von hauptsächlich aus Cu-Verbindungen und gegebenenfalls auch von Cu-Metall bestehenden Katalysatoren nach den einschlägigen Verfahren des Standes der Technik (wie anfangs beschrieben) hergestellt wurde. Vorzugsweise wird als Ausgangsmaterial ein in Gegenwart von hauptsächlich nur Cu-I-chlorid und gegebenenfalls elementarem Cu als Katalysator nach dem Verfahren der US-PS 3 943 175 (ohne die dortige Reinigung) hergestelltes Roh-TAD verwendet. Das Produkt kann in dem Zustand, in welchen es beim Herstellungsprozess anfällt, also durchaus in noch mutterlaugenfeuchter Form, eingesetzt werden. Bevorzugt ist allerdings der Einsatz eines Roh-TAD, das nach dem Herstellungsprozess von der Mutterlauge über eine Drucknutsche oder unter Verwendung einer (Schäl-) Zentrifuge abgetrennt wurde.

Der Cu-Gehalt des nach den erwähnten bekannten Verfahren erhaltenen und entsprechend abgetrennten Roh-TAD liegt im allgemeinen zwischen etwa 3 und 6 (Gew.-)%. Für den Erfolg des erfindungsgemässen Verfahrens ist es günstig, dass das dem Verfahren zugeführte Roh-TAD nach seiner Isolierung aus dem Herstellungsprozess (Abfiltration, Abnutschen, Zentrifugieren etc.) nicht vorher mit Wasser ausgewaschen wird.

Die Behandlung des Roh-TAD nach seiner Isolierung aus dem Herstellungsprozess mit einer wässrigen $NH_3$-Lösung gemäss Stufe a) des erfindungsgemässen Verfahrens geschieht zweckmässig durch Auswaschen mit wässrigem Ammoniak bei Raumtemperatur und Normaldruck. In diesem Fall wird im allgemeinen eine wässrige $NH_3$-Lösung einer $NH_3$-Konzentration zwischen etwa 10 und 28 (Gew.-)%, bevorzugt von etwa 20 bis etwa 28 (Gew.-)% verwendet. Wenn man die Behandlung unter Druck durchführt (was prinzipiell möglich ist), kann die $NH_3$-Konzentration auch über etwa 28 (Gew.-)% liegen.

Für die Arbeitsweise bei Raumtemperatur sind im allgemeinen pro 100 g Roh-TAD Mengen an wässriger $NH_3$-Lösung des Konzentrationsbereiches ca. 10 bis 28% von etwa 50 bis 1000 ml ausreichend; in dem bevorzugten Konzentrationsbereich der wässrigen $NH_3$-Lösung von etwa 20 bis 28% sind etwa 20 bis 28% sind etwa 200 bis 400 ml erforderlich.

Nach der erfindungsgemässen Behandlung mit wässriger $NH_3$-Lösung wird das TAD normalerweise mit Wasser neutral gewaschen. Der Cu-Gehalt des so behandelten Roh-TAD liegt zumindest in der Grössenordnung des nach dem Verfahren der US-PS 3 943 175 über das Sulfat gereinigten Produktes (etwa 0,6 bis 0,9%), meist jedoch darunter. Bei sorgfältiger Verfahrensweise sind Cu-Gehalte sogar um 0,2% und darunter erzielbar.

Für manche Einsatzzwecke des TAD kann die Senkung des Cu-Gehaltes auf Werte bis etwa 0,2% ausreichen. In diesen Fällen ist dann keine weitere Behandlung mehr erforderlich.

Im allgemeinen stören aber Cu-Gehalte selbst von etwa 0,2% noch erheblich. In diesen Fällen ist dann noch Stufe b) des erfindungsgemässen Verfahrens anzuschliessen. Diese Stufe besteht in einem Umlösen des gemäss Stufe a) gereinigten Roh-TAD aus Wasser in Gegenwart von Adsorbentien sowie noch eines wasserlöslichen Reduktionsmittels. Dieses Umlösen wird im Prinzip wie in der US-PS 3 943 175 ohne den Zusatz eines wasserlöslichen Reduktionsmittels beschrieben durchgeführt. Das TAD aus Stufe a) des erfindungsgemässen Verfahrens wird also demnach in siedendem Wasser gelöst. Der Lösung werden übliche Adsorbentien wie z.B. Aktivkohle, Diatomeenerde, Kieselgur etc. – vorzugsweise eine Aktivkohle der Oberfläche etwa 1100 bis 1200 $m^2/g$ – zugesetzt. Die Menge des Adsorbens beträgt normalerweise zwischen etwa 10 und 30 (Gew.-)%, vorzugsweise zwischen etwa 15 und 25 (Gew.-)% bezogen auf Roh-TAD (trocken).

Erfindungsgemäss wird dieser Lösung zusätzlich noch ein wasserlösliches Reduktionsmittel wie z.B. ein Alkalisulfit, Alkalisulfid, Alkalidithionit etc. – vorzugsweise Natriumdithionit $Na_2S_2O_4$ – zugesetzt. Wenn ein Dithionit verwendet wird, ist es zweckmässig, davon etwa 2 bis 10 (Gew.-)%, vorzugsweise etwa 2 bis 3 (Gew.-)%, bezogen auf Roh-TAD (trocken) einzusetzen.

Es ist vorteilhaft, die wässrige Lösung bzw. Suspension von Stufe b) des erfindungsgemässen Verfahrens kurze Zeit – vorzugsweise etwa 1 bis 1,5 Stunden – am Rückfluss zu kochen. Danach wird die Lösung bzw. Suspension filtriert und das Filtrat auf etwa Raumtemperatur kalt gerührt. Dabei fällt eine nahezu farbloses bis schwach sandfarbenes reines TAD mit einem Cu-Gehalt zumindest in der Grössenordnung des auch nach dem Verfahren der US-PS 3 943 175 erhaltenen TAD (etwa 0,01 bis 0,07% Cu), meist jedoch darunter, an. Bei sorgfältiger Verfahrensführung können Cu-Gehalte von etwa 0,005 (Gew.-)% und darunter erzielt werden. Der Fp- des Produktes liegt bei 177 bis 180°C (theoretisch: 179 bis 182°C). Ausbeuten an reinem TAD bis etwa 77% d.Th., bezogen auf das Ausgangs-Roh-TAD (trokken) sind so ohne weiteres erzielbar. Wegen der hohen Sauerstoffempfindlichkeit des TAD sind zwecks Vermeidung einer Ausbeuteminderung sämtliche Operationen des erfindungsgemässen Verfahrens unter Sauerstoffausschluss – also unter einem Inertgas (vorzugsweise Stickstoff) – vorzunehmen.

Hauptsächlich wegen der gegenüber den einschlägigen Verfahren des Standes der Technik (US-PS 3 943 175) um etwa 25% höheren Ausbeute sowie auch der einfacheren Verfahrensführung stellt das erfindungsgemässe Reinigungsverfahren einen erheblichen Fortschritt auf diesem Gebiet dar.

Das folgende Beispiel soll der weiteren Erläuterung der Erfindung dienen. Das daran anschliessende Vergleichsbeispiel zeigt, dass die Durchführung der erfindungsgemässen Behandlung des Roh-TAD mit wässriger $NH_3$-Lösung vor dem Neutralwaschen mit Wasser kritisch ist.

Die in dem (Erfindungs-) und dem Vergleichsbeispiel angegebenen Teile sind Gewichtsteile. Sämtliche Operationen bei der Herstellung und der Reinigung des TAD wurden unter Stickstoff vorgenommen.

Beispiel

Herstellung von TAD (nach dem Stand der Technik):

In einem 2 l V4A Autoklav werden 222 Teile DCB, feucht ($H_2O$-Gehalt 19,7%), 27,7 Teile $Cu_2Cl_2$, 8,9 Teile Cu-Bronze bzw. Cu-Pulver und 1179 Teile 25%iger, wässriger $NH_3$-Lösung vorgelegt und die im Autoklav befindliche Luft durch zweimaliges Aufdrücken und Entspannen von 20 bis 25 bar $N_2$ entfernt. Anschliessend werden noch 94 Teile $NH_3$, flüssig ($d_{20}$: 0,61) eingedrückt und der Autoklav 7 h auf 200°C erhitzt, wobei der Druck von anfänglich 55 bar auf ca. 50 bar abfällt. Nach beendeter Reaktionsdauer wird der Autoklavinhalt nach dem Abkühlen auf 25°C und dem Entspannen über eine Drucknutsche abgedrückt. Ausbeute: 200 g Roh-TAD (feucht) = 141 g (trokken)

a) erfindungsgemässe Behandlung des Roh-TAD mit wässriger $NH_3$-Lösung:

Das mutterlaugenfeuchte Roh-TAD (200 g) wird nacheinander mit ca. 500 Teilen ca. 25 %iger wässriger $NH_3$-Lösung in 2 Portionen zu je 250 Teilen und mit 1000 Teilen $H_2O$ in jeweils 2 Portionen gewaschen.

Ausbeute an TAD (trocken): 136 g = 96,5% d.Th., bezogen auf Roh-TAD;

| | |
|---|---|
| Cu-Gehalt: | ca. 0,7% |
| Fp.: | 175–177°C |

Bei Verwendung einer Schälzentrifuge anstelle der Drucknutsche zwecks Isolierung des Roh-TAD werden – bis auf den Cu-Gehalt (hier: ca. 0,2%) – praktisch die gleichen Werte erhalten.

b) erfindungsgemässes Umlösen:

Zur Umlösung des angefallenen TAD werden in einem 6 l Rührkolben 4 l Wasser, 30 Teile Aktivkohle (vom Typ Carboraffin C bzw. P der Fa. Lurgi) und 199 Teile TAD (aus Stufe a)) mit einem $H_2O$-Gehalt von ca. 30% vorgelegt, die Luft durch zweimaliges Evakuieren und Belüften mit $N_2$ entfernt, dann noch 3 Teile $Na_2S_2O_4$ zugesetzt und 1 bis 1,5 Stunden lang unter Rühren zum Sieden erhitzt. Anschliessend wird die Aktivkohle heiss abgedrückt, mit ca. 150 ml heissem Wasser nachgewaschen und das Filtrat nach Zugabe von 1 Teil $Na_2S_2O_4$ auf ca. 25°C kaltgerührt. Das auskristallisierte TAD, rein, wird unter $N_2$ abgesaugt und im Vakuum getrocknet. Das anfallende TAD, rein, ist weiss bis schwach sandfarben gefärbt. Ausbeute an TAD, rein: 107 g = 75,9% d.Th., bezogen auf Roh-TAD, bzw. 71,3% d.Th., bezogen auf DCB.

| | |
|---|---|
| Cu-Gehalt: | $\leqq$ 0,005% |
| Fp.: | 177–178°C |

Vergleichsbeispiel:

Zum Vergleich wird wie in Beispiel 1 verfahren, jedoch das abgedrückte und mit 25 %iger wässriger $NH_3$-Lösung gewaschene TAD (roh) in Abwesenheit von $Na_2S_2O_4$ umgelöst. Das anfallende TAD (rein) ist grau bis dunkelgrau gefärbt.

Ausbeute rein: 107 g = 75,9% d.Th., bezogen auf TAD roh, bzw. 71,3% d.Th., bezogen auf DCB.

Cu-Gehalt: 0,006%
Fp.: 177–178°C

**Patentansprüche**

1. Verfahren zur Reinigung von rohem 3,4,3′,4′-Tetraaminodiphenyl (TAD), welches durch Ammonolyse von 3,3′-Dichlorbenzidin (DCB) in Gegenwart hauptsächlich von Cu-Katalysatoren hergestellt wurde,

a) durch Behandlung des Roh-TAD mit einer wässrigen Lösung sowie gegebenenfalls
b) Umlösen des so behandelten TAD aus Wasser in Gegenwart von Adsorbentien,

dadurch gekennzeichnet, dass man

a) das Roh-TAD nach seiner Isolierung aus dem Herstellungsprozess mit einer $NH_3$-Lösung behandelt und dass man
b) beim Umlösen des so behandelten TAD aus Wasser in Gegenwart von Adsorbentien noch ein wasserlösliches Reduktionsmittel zusetzt.

2. Verfahren nach Anspruch 1, dadurch gekennzeichnet, dass man als wässrige $NH_3$-Lösung in Stufe a) eine etwa 10 bis 28 gew.- %ige, vorzugsweise etwa 20 bis 28 gew.- %ige wässrige $NH_3$-Lösung verwendet.

3. Verfahren nach einem der Ansprüche 1 bis 2, dadurch gekennzeichnet, dass man als wasserlösliches Reduktionsmittel in Stufe b) ein Alkalidithionit, vorzugsweise Na-dithionit verwendet.

**Claims:**

1. A process for the purification of crude 3,4,3′,4′-tetraaminodiphenyl (TAD) prepared by ammonolysis of 3,3′-dichlorbenzidine (DCB) in the presence of mainly Cu catalysts,

a) by treatment of crude TAD with an aqueous solution and, if desired,
b) dissolving and reprecipitating TAD thus treated in water in the presence of adsorbents,

characterized in

a) treating crude TAD after its isolation from the preparation process with an aqueous $NH_3$ solution and
b) also adding a water-soluble reducing agent when TAD thus treated is dissolved and reprecipitated in water in the presence of adsorbents.

2. The process as claimed in claim 1, characterized in using as aqueous $NH_3$ solution in stage a) an approximately 10 to 28% by weight strength, preferably about 20 to 28% by weight strength, aqueous $NH_3$ solution.

3. The process as claimed in anyone of claims 1 to 2, characterized in using as a water-soluble reducing agent in stage b) an alkali metal dithionite, preferably Na dithionite.

**Revendications**

1. Procédé de purification de tétraamino-3,4,3′,4′biphényle (TAB) brut qui a été préparé par ammonolyse de la dichloro-3,3′ benzidine (DCB) en présence de catalyseurs essentiellement constitués de Cu, procédé selon lequel:

a) on traite le TAB brut par une solution aqueuse et éventuellement
b) on recristallise le TAB ainsi traité, dans de l'eau, en présence d'adsorbants,

et qui est caractérisé en ce que:

a) on traite le TAB brut, après l'avoir isolé à partir de son mélange de préparation, par une solution d'$NH_3$ et
b) lors de la recristallisation du TAB ainsi traité, dans de l'eau, en présence d'adsorbants, on ajoute un réducteur soluble dans l'eau.

2. Procédé selon la revendication 1 caractérisé en ce qu'on utilise, dans l'étape a), comme solution aqueuse d'$NH_3$, une solution ammoniacale aqueuse d'une concentration comprise entre environ 10 et 28% en poids, de préférence entre environ 20 à 28% en poids.

3. Procédé selon l'une des revendications 1 et 2, caractérisé en ce qu'on utilise comme réducteur hydrosoluble, dans le stade b), un dithionite de métal alcalin, de préférence le dithionite de sodium.